# EUROPÄISCHE PATENTSCHRIFT

(11) **EP 0 903 109 B1**
(45) Veröffentlichungstag und Bekanntmachung des Hinweises auf die Patenterteilung: **16.07.2003**
(21) Anmeldenummer: 98890252.4
(22) Anmeldetag: 26.08.1998
(51) Int. Cl.: A61B 17/04

(54) **Chirurgische Nahtzange**
Suturing device
Appareil pour la pose de sutures

(30) Priorität: 09.09.1997 AT 151797
(43) Veröffentlichungstag der Anmeldung: 24.03.1999
(73) Patentinhaber: Fuchs, Werner, 2443 Loretto (AT)
(72) Erfinder: Fuchs, Werner, Ing., 2443 Loretto (AT); Boszotta, Harald Dr., 2491 Steinbrunn; Neue Siedlung (AT)
(74) Vertreter: Puchberger, Peter, Dipl.-Ing.

(56) Entgegenhaltungen:
- US-A- 5 397 325
- US-A- 5 522 820

## Beschreibung

Die Erfindung betrifft eine chirurgische Nahtzange mit einem beweglichen Nähdorn und wenigstens einer Zangenbranche mit einer Fadenführung, wobei eine feststehende und eine bewegliche Zangenbranche vorhanden ist.

Chirurgische Nahtzangen werden beispielweise in der arthroskopischen Chirurgie verwendet, wobei die Nahtzange durch relativ enge Körperöffnungen bzw. durch eine Kanüle eingeführt und betätigt werden muß. So ist es beispielsweise im Bereich der Schulterchirurgie erforderlich, eine arthroskopische Refixierung der eingerissenen Rotatorenmanschette vorzunehmen. Dabei wird durch eine Zugangskanüle ein Faden (oder auch mehrere Fäden) oder eine Fadenschlinge durch die gerissene Sehne gezogen und mittels der Fadenschlinge wird die Rotatorenmanschette wieder am Knochen des Oberarm fixiert. Die Verankerung oder Fixierung am Knochen erfolgt durch Bohrkanäle oder mittels Fadenanker oder Fadendübel gemäß herkömmlicher Operationstechniken.

Die eingangs beschriebene Nahtzange ist z.B. durch die US-PS 5 522 820 der Firma Arthrotek bekannt geworden. Diese bekannte Nahtzange (siehe Fig.1) weist einen mit der beweglichen Zangenbranche fix verbundenen Nähdorn auf, der durch die Zangenbewegung quer zur Zangenlängsache durch die Sehne gedrückt wird. Nach dem Durchstoßen der Sehne fängt der Nähdorn den an einer zweiteiligen unteren feststehenden Zangenbranche in Nuten vorgelegten Faden. Durch nachfolgendes öffnen der Nahtzange wird der Nähdorn mit der Fadenschlinge durch das Loch in der Sehne zurückgezogen. Beim Herausziehen der geöffneten Nahtzange kommt die Fadenschlinge zur Fixierungsstelle am Knochen und kann dort fixiert werden.

Der Nachteil dieser bekannten Konstruktion liegt darin, daß der Nähdorn starr an der beweglichen Zangenbranche fixiert ist. Zum Einführen der Nahtzange ist eine Kanüle mit großer Bohrung erforderlich, zB. mit einem Durchmesser von 12 mm. Weiters muß die Nahtzange zum Umfassen eines ausreichend starken Sehnenanteiles auf eine Aussenabmessung von 18 bis 20 mm geöffnet werden. Zum Erfassen der Sehne muß diese bereits durchgestochen sein. Bei stark zurückgezogenen Sehnenanteilen kann es geschehen, daß diese nicht weit genug erfaßt oder nicht an der vorgesehnen Stelle erfaßt werden, wobei die arthroskopische Sicht überdies schlecht ist.

Die DE 24 47 719 A1 zeigt eine Nahtzange, bei der in nachteiliger Weise der Nähdorn gabelförmig und eine Klemmvorrichtigung für die Übergabe des Fadens an die bewegte Zangenbranche vorgesehen sein muß.

Die geschilderten Nachteile sollen mit der erfindungsgemäßen Nahtzange vermieden, und es soll eine Nahtzange mit verbesserter Funktionsfähigkeit vorgesehen werden.

Die erfindungsgemäße chirurgische Nahtzange ist dadurch gekennzeichnet, daß die bewegliche Zangenbranche durch ein erstes Zug-Schubelement bewegbar ist, und daß durch ein zweites Zug-Schubelement der Nähdorn in Richtung der Längsachse der Nahtzange bewegbar und zur beweglichen Zangenbranche hin auslenkbar ist, um den von der beweglichen Zangenbranche geführten Faden mit einem Fanghaken zu hintergreifen.

Weitere vorteilhafte Merkmale sind den Unteransprüchen, der nachfolgenden Beschreibung und den Zeichnungen zu entnehmen.

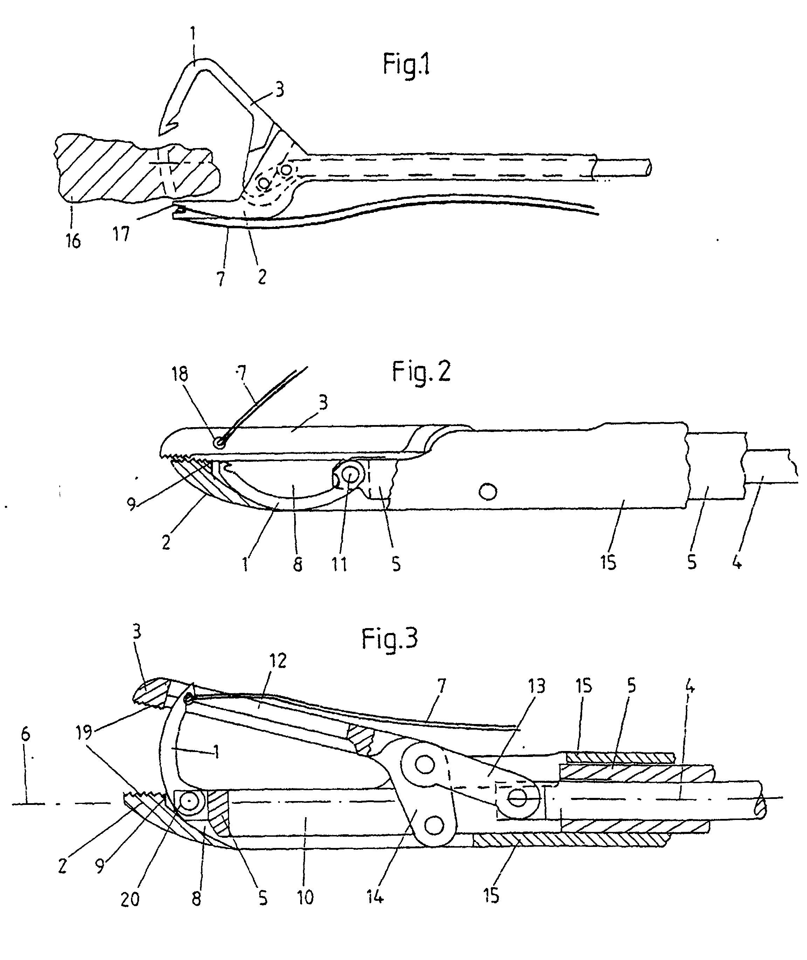

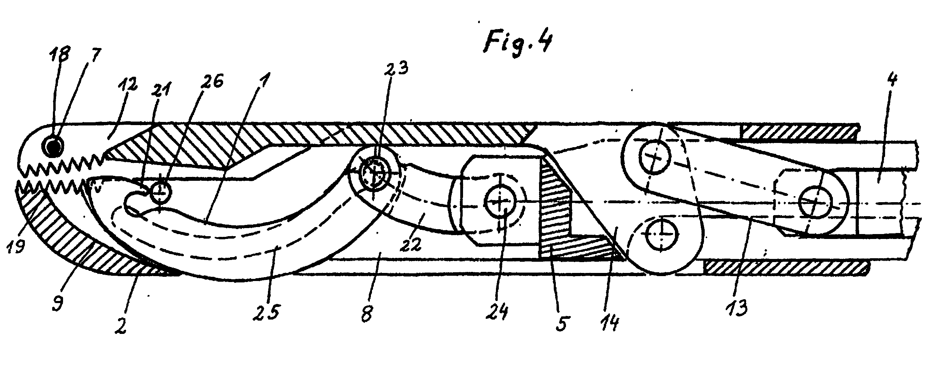

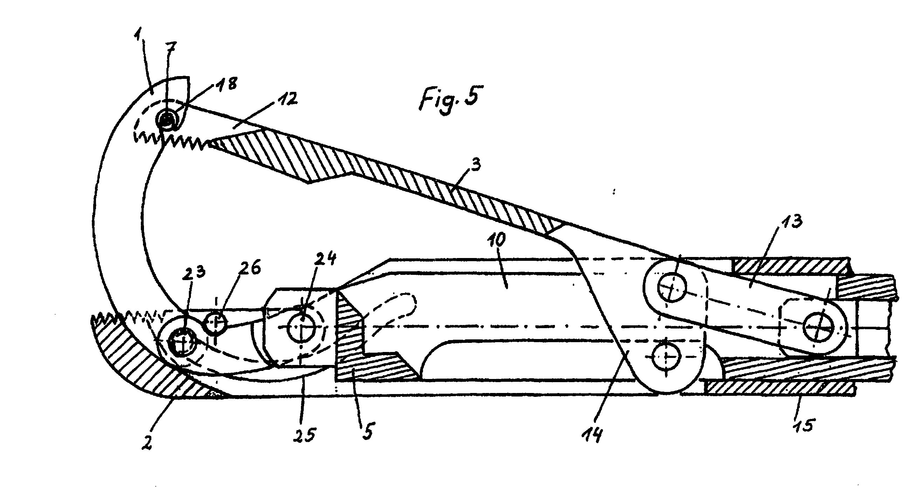

Fig.1 zeigt schematisch eine Nahtzange gemäß Stand der Technik. Fig.2 ist eine teilweise geschnitte Seitenansicht des Kopfes der erfindungsgemäßen Nahtzange in einer ersten Funktionsstellung und Fig.3 die Nahtzange in einer weiteren Funktionsstellung im Schnitt entlang der Längsachse der Nahtzange. Die Fig. 4 und 5 zeigen schematisch eine weitere vorteilhafte Ausgestaltung der Erfindung in zwei FunktionsStellungen.

Gemäß Fig.1 weist die bekannte Nahtzange einen mit der beweglichen Zangenhranche 3 fix verbundenen Nähdorn 1 auf, der durch die Zangenbewegung quer zur Zangenlängsachse durch die Sehne 16 gedrückt wird. In Nuten 17 der zweigeteilten feststehenden Zangenbranche 2 ist der Faden 7 vorgelegt, wird vom Nähdorn 1 hintergriffen und durch Öffnen der Zange wird die Fadenschlinge durch die Sehne 16 durchgezogen. Dabei treten die eingangs geschilderten Nachteile auf. In Fig.1 und auch in den nachfolgenden Fig.2 und 3 betreffend die erfindungsgemäße Konstruktion sind die Handhaben für die Nahtzange nicht dargestellt, da sie nicht Gegenstand der vorliegende Erfindung sind. Beispielsweise kann die Handhabe einen Griff in Art eines Pistolengriffes oder Scherengriffes mit angelenkten Betätigungshebeln umfassen.

Die Fig.2 zeigt den Kopf der erfindungsgemäßen Nahtzange in teilweise geschnittener Darstellung. Die Zange ist in geschlossenem Zustand. Der Durchmesser ist gering und die Nahtzange kann somit leicht durch enge Körperöffnungen oder Kanülen eingeführt werden. Weiters ragen keine Konstruktionsteile hervor, die der Bewegung hinderlich sein können. In der Praxis kann der maximale Aussendurchmesser 7 mm sein und die Zugangsöffnung kann entsprechend klein gehalten werden.

Die Fig.3 zeigt die Nahtzange in teilweise geöffnetem Zustand in jener Position, in der der Nähdorn den Faden fängt.

Grundsätzlich erfolgt gemäß Erfindung die Bewegung des Nähdorns getrennt und unabhängig von der Bewegung der Zangenbranchen. Damit ist es möglich, vorerst mit den Zangenbranchen den Gewebeteil richtig zu fassen, und erforderlichenfalls mehrmals zuzugreifen, und bei richtiger Positionierung den Nähdorn durch den Gewebeteil durchzustoßen und den Faden zu holen, sowie als Schlinge wieder zurückzuziehen.

Der Schaft der Nahtzange besteht aus einem äußeren Hüllrohr 15, das im Kopfbereich der Nahtzange in die feststehende Zangenbranche 2 übergeht. Die feststehende Zangenbranche 2 weist eine Führungsnut 8 auf, in der der Nähdorn 1 verschiebbar ist. Der Nähdorn 1 ist über das Gelenk 11 am zweiten Zugschubelement 5 angelenkt.

Die Führungsnut 8 weist eine Auflaufkante 9 auf, durch die der Nähdorn 1 beim Vorschub des zweiten Zug-Schubelements 5 in die Richtung quer zur Längsachse 6 der Nahtzange und zur beweglichen Zangenbranche 3 hin ausgelenkt wird, wie dies in Fig.3 dargestellt ist.

Die bewegliche Zangenbranche 3 ist über eine Gelenkgabel 14 an der feststehenden Zangenbranche 2 angelenkt. Über einen Zug-Schubschenkel 13 ist die bewegliche Zangenbranche 3 mit dem ersten Zug-Schubelement 4 verbunden. Der Schwenkbereich der beweglichen Zangenbranche 3 ist sehr groß. Im vorderen Teil ist die bewegliche Zangenbranche mit einem Längsschlitz 12 versehen, damit der Nähdorn 1 durchtreten und den Faden 7 holen kann. Der Faden 7 ist in einer Fadenbohrung 18 geführt.

Die ersten und zweiten Zug-Schubelemente 4,5 sind in dem Hüllrohr 15 aufgenommen. Das zweite Zug-Schubelement 5 ist in der dargestellten Variante als Rohr ausgebildet und nimmt das erste Zug-Schubelement in Form einer Stange auf. Das Rohr des zweiten Zug-Schubelementes 5 weist im Bereich des Kopfes der Nahtzange einen Längsschlitz 10 auf, um Platz für den Durchtritt des Zug-Schubschenkels 13 zu bieten, der innerhalb der Gelenkgabel 14 angeordnet ist.

Die Zangenbranchen können nach vorne hin auch offen ausgeführt sein. Bevorzugt besitzen beide an der Innenseite eine Riffelung 19, um den Gewebeteil besser fassen zu können. Anstelle der Fadenbohrung 18 kann auch eine schlitzförmige Fadenführung vorliegen.

Die Rückbewegung des Nähdorns kann durch eine Feder 20 unterstützt werden, wie sie in Fig.3 beispielsweise eingezeichnet ist.

Beim wird die Sehne zuerst mit dem durch die beiden Zangenbranchen gebildeten Zangenbereich gefaßt und in den Sichtbereich des Arthroskops gezogen. Wenn die Position für die Naht geeignet ist, wird durch die Zangenbetätigung das zweite Zug-Schubelement nach vorne geschoben, wodurch der Nähdorn ebenfalls nach vorne gedrückt wird. Der gelenkig angebrachte Nähdorn 1 wird durch die Auflaufkante 9 der unteren feststehenden Zangenbranche 2 nach oben gedrückt. Dabei perforiert er die Sehne und tritt in den Längsschlitz 12 der beweglichen Zangenbranche ein, wo er unter guter arthroskopischer Sicht den vorgelegten Faden 7 erfaßt. Bei noch immer geschlossener Zange wird der Faden mit dem Nähdorn zurückgezogen, wobei sich dieser Nähdorn wieder in die Führungsnut 8 der feststehenden Zangenbranche zurücklegt und den Faden mitnimmt. Daraufhin wird die Zange leicht geöffnet und zurückgezogen, wobei die gebildete Fadenschlinge mitgezogen wird.

Bei der Ausführung gemäß den Figuren 4 und 5 ist zwischen dem Nähdorn 1 und dem zweiten Zug-Schubelement 5 ein Zwischenglied 22 angeordnet. Das Zwischenglied 22 ist über eine hintere Schwenkachse 24 mit dem zweiten Zugschubelement 5 und über eine vordere Schwenkachse 23 mit dem Nähdorn 1 gelenkig verbunden. Die vordere Schwenkachse 23 ist weiteres in einer kurvenförmigen Führungskulisse 25 der Führungsnut 8 der feststehenden Zangenbranche 2 geführt. Die Fig.4 zeigt die Nahtzange in geschlossenem Zustand, wobei der Nähdorn und das Zwischenglied 22 völlig in der Führungsnut 8 liegt.

Die Fig.5 zeigt die Funktionsstellung bei geöffneter beweglicher Zangenbranche 3 und dem zur Gänze ausgefahrenen Nähdorn 1. In der feststehenden Zangenbranche 2 sitzt ein die Führungsnut 8 übergreifender Führungsstift 26, der den Nähdorn 1 und das Zwischenglied 22 bei deren Bewegungen führt. Eine zusätzliche Führung erfolgt durch die Führungskulisse 25, die einen S-förmigen Verlauf hat.

Die bewegliche Zangenbranche 3 weist in dieser Ausführungsform den Längsschlitz 12 für den Durchtritt des Nähdorns an ihrer Spitze auf, die sonst gabelförmig ist, sodaß der Nähdorn den in der Fadenbohrung 18 geführten Faden mit dem Fanghaken 21 hintergreifen kann.

## Patentansprüche

1. Chirurgische Nahtzange mit einem beweglichen Nähdorn und wenigstens einer Zangenbranche mit einer Fadenführung, wobei die Nahtzange eine feststehende und eine bewegliche Zangenbranche (2,3) aufweist, **dadurch gekennzeichnet, daß** die bewegliche Zangenbranche (3) durch ein erstes Zug-Schubelement (4) bewegbar ist, und daß durch ein zweites Zug-Schubelement (5) der Nähdorn (1) in Richtung der Längsachse (6) der Nahtzange bewegbar und zur beweglichen Zangenbranche (3) hin auslenkbar ist, um den von der beweglichen Zangenbranche (3) geführten Faden (7) mit einem Fanghaken (21) zu hintergreifen.

2. Nahtzange nach Anspruch 1, **dadurch gekennzeichnet, daß** der Nähdorn (1) am zweiten Zug-Schubelement (5) angelenkt ist und in einer Führungsnut (8) der festehenden Zangenbranche (2) der Länge nach verschiebbar ist und daß die Führungsnut (8) eine Auflaufkante (9) aufweist, durch die der Nähdorn (1) bei Vorschub in eine Richtung quer zur Längsachse (6) der Nahtzange und zur beweglichen Zangenbranche (3) hin ausgelenkt wird.

3. Nahtzange nach Anspruch 1 oder 2, **dadurch gekennzeichnet, daß** zwischen dem zweiten Zug-Schubelement (5) und dem Nähdorn (1) ein Zwischenglied (22) angeordnet ist, dessen vordere Schwenkachse (23) in einer kurvenförmigen Führungskulisse (25) der Führungsnut (8) der feststehenden Zangenbranche (2) geführt ist.

4. Nahtzange nach Anspruch 3, **dadurch gekennzeichnet, daß** die Führungskulisse (25) einen S-förmigen Verlauf aufweist.

5. Nahtzange nach einem der Ansprüche 1 bis 4, **dadurch gekennzeichnet, daß** an der feststehenden Zangenbranche (2) ein die Führungsnut (8) übergreifender Führungsstift (26) vorgesehen ist.

6. Nahtzange nach einem der Ansprüche 1 bis 5, **dadurch gekennzeichnet, daß** die bewegliche Zangenbranche (3) einen Längsschlitz (12) für den Durchtritt des Nähdorns (1) aufweist.

7. Nahtzange nach den Ansprüchen 1 bis 6, **dadurch gekennzeichnet, daß** das zweite Zug-Schubelement (5) ein Rohr ist, an dessen einem Ende das Gelenk (11) des Nähdornes (1) oder des Zwischengliedes (22) angeordnet ist und daß das Rohr im Bereich bis zum Gelenk (11) durch einen Längsschlitz (10) für den Durchtritt eines Zug-Schubschenkels (13) zum Verschwenken der beweglichen Zangenbranche (3) freigestellt ist.

8. Nahtzange nach Anspruch 7, **dadurch gekennzeichnet, daß** der Zug-Schubschenkel (13) an dem ersten Zug-Schubelement (4), bevorzugt einer Stange, angelenkt ist.

9. Nahtzange nach einem der vorhergehenden Ansprüche 1 bis 8, **dadurch gekennzeichnet, daß** die bewegliche Zangenbranche (3) über eine Gelenkgabel (14) an der feststehenden Zangenbranche (2) angelenkt ist.

10. Nahtzange nach einem der vorhergehenden Ansprüche 1 bis 9, **dadurch gekennzeichnet, daß** die feststehende Zangenbranche (2) mit einem Hüllrohr (15) verbunden ist, welches die ersten und zweiten Zug-Schubelemente (4,5) der Länge nach umhüllt.

## Claims

1. Surgical suture forceps having a movable suturing spike and at least one branch of the forceps that has a thread guide, wherein the suture forceps has a fixed branch and a movable branch (2, 3) of the forceps, **characterised in that** the movable branch (3) of the forceps can be moved by a first pull-push element (4), and that the suturing spike (1) can be moved in the direction of the longitudinal axis (6) of the suture forceps by a second pull-push element (5) and deflected towards the movable branch (3) of the forceps, in order to engage, with a catching hook (21), behind the thread (7) guided by the movable branch (3) of the forceps.

2. Suture forceps according to claim 1, **characterised in that** the suturing spike (1) is articulated on the second pull-push element (5) and can be displaced lengthwise in a guide groove (8) in the fixed branch (2) of the forceps, and that the guide groove (8) has a run-up edge (9) by which the suturing spike (1) is deflected, when advanced, into a direction transverse to the longitudinal axis (6) of the suture forceps and towards the movable branch (3) of the forceps.

3. Suture forceps according to claim 1 or 2, **characterised in that** an intermediate member (22), whose front pivoting spindle (23) is guided in a curviform guide gate (25) in the guide groove (8) of the fixed branch (2) of the forceps, is disposed between the second pull-push element (5) and the suturing spike (1).

4. Suture forceps according to claim 3, **characterised in that** the guide gate (25) has an S-shaped path.

5. Suture forceps according to one of claims 1 to 4, **characterised in that** a guide pin (26) which crosses the guide groove (8) is provided on the fixed branch (2) of the forceps.

6. Suture forceps according to one of claims 1 to 5, **characterised in that** the movable branch (3) of the forceps has a longitudinal slot (12) for the passage of the suturing spike (1).

7. Suture forceps according to claims 1 to 6, **characterised in that** the second pull-push element (5) is a tube, at one end of which the joint (11) of the suturing spike (1) or of the intermediate member (22) is disposed, and that the tube is freed, in the region up to the joint (11), by a longitudinal slit (10) for the passage of a pull-push leg (13) for swivelling the movable branch (3) of the forceps.

8. Suture forceps according to claim 7, **characterised in that** the pull-push leg (13) is articulated on the first pull-push element (4), which is preferably a bar.

9. Suture forceps according to one of the preceding claims 1 to 8, **characterised in that** the movable branch (3) of the forceps is articulated on the fixed branch (2) of the forceps via a fork-type joint (14).

10. Suture forceps according to one of the preceding claims 1 to 9, **characterised in that** the fixed branch (2) of the forceps is connected to an enveloping tube (15) which envelops the first and second pull-push elements (4, 5) lengthwise.

## Revendications

1. Dispositif pour la pose de sutures comportant un mandrin de suture mobile et au moins une branche présentant un guide-fil, le dispositif pour la pose de sutures présentant ainsi une branche fixe et une branche mobile (2, 3), **caractérisé en ce que** la branche mobile (3) est actionnée par un premier élément de traction/poussée (4), et **en ce qu'**un second élément de traction/poussée (5) autorise le déplacement du mandrin de suture (1) dans le sens de l'axe longitudinal (6) du dispositif pour la pose de sutures et son retour vers la branche mobile (3) afin de saisir le fil (7) guidé par la branche mobile (3) au moyen d'un crochet d'arrêt (21).

2. Dispositif selon la revendication 1, **caractérisé en ce que** le mandrin de suture (1) est articulée au second élément de traction/poussée (5), et peut coulisser jusqu'à une rainure de guidage (8) de la branche fixe (2) dans le sens de la longueur, et **en ce que** la rainure de guidage (8) présente un bord (9) d'attaque, par lequel le mandrin de suture (1) est dirigé lors de l'avance dans une direction transversale à l'axe longitudinal (6) du dispositif pour la pose de sutures et vers la branche mobile (3).

3. Dispositif selon la revendication 1 ou 2, **caractérisé en ce qu'**il est disposé entre le second élément de traction/poussée (5) et le mandrin de suture (1) un élément intermédiaire (22), dont l'axe de pivotement avant (23) est guidé dans une coulisse de guidage courbée (25) de la rainure de guidage (8) de la branche fixe (2).

4. Dispositif selon la revendication 3, **caractérisé en ce que** la coulisse de guidage (25) présente une forme en S.

5. Dispositif selon l'une quelconque des revendications 1 à 4, **caractérisé en ce qu'**une tige de guidage (26) recouvrant la rainure de guidage (8) est prévue sur la branche fixe (2).

6. Dispositif selon l'une quelconque des revendications 1 à 5, **caractérisé en ce que** la branche mobile (3) présente une fente longitudinale (12) permettant le passage du mandrin de suture (1).

7. Dispositif selon les revendications 1 à 6, **caractérisé en ce que** le second élément de traction/poussée (5) est un tube, à l'une des extrémités duquel est située l'articulation (11) du mandrin de suture (1) ou de l'élément intermédiaire (22), et **en ce que** le tube présente dans la partie allant jusqu'à l'articulation (11) une fente longitudinale (10) permettant le passage d'une biellette de traction/poussée (13) assurant le pivotement de la branche mobile (3).

8. Dispositif selon la revendication 7, **caractérisé en ce que** la biellette de traction/poussée (13) est articulée au premier élément de traction/poussée (4), de préférence par un axe.

9. Dispositif selon l'une quelconque des revendications précédentes, **caractérisé en ce que** la branche mobile (3) est articulé à la branche fixe (2) au moyen d'une fourche (14).

10. Dispositif selon l'une quelconque des revendications précédentes, **caractérisé en ce que** la branche fixe (2) est reliée à un tube enveloppe (15), qui enveloppe le premier et le second élément de traction/poussée (4, 5) dans le sens de la longueur.
